# EUROPEAN PATENT APPLICATION

(11) **EP 3 949 749 A1**
(43) Date of publication of application: **09.02.2022**
(21) Application number: 20212994.6
(22) Date of filing: 10.12.2020
(51) Int. Cl.: A23K 20/10, A23K 20/142, A23K 20/121, A23K 50/10, A23K 50/30, A23K 50/60, A23L 33/105, A23L 33/175, A61K 31/198, A61K 31/353

(54) **AGENT FOR IMPROVING INTESTINAL MICROFLORA OF LIVESTOCK**

(30) Priority: 07.08.2020 JP 2020135089
(71) Applicant: AJINOMOTO CO., INC., Chuo-ku Tokyo 104-8315 (JP)
(72) Inventor: CHALVON, Demersey Tristan, Kanagawa, 210-8681 (JP); SUSA, Yuko, Kanagawa, 210-8681 (JP); SATO, Wataru, Kanagawa, 210-8681 (JP); BANNAI, Makoto, Kanagawa, 210-8681 (JP); OOTA, Yasuhiro, Kanagawa, 210-8681 (JP)
(74) Representative: Strehl Schübel-Hopf & Partner

(57) **Abstract**

[Object]

An object of the present invention is to increase lactic acid bacteria in the body of livestock by a simple method.

[Means for Solution]

The present invention relates to an agent for improving the intestinal microflora of livestock, containing arginine and tannin as active ingredients.

## Description

### [Technical Field]

The present invention relates to an agent for improving the intestinal microflora of livestock. More particularly, the invention relates to an agent for improving the intestinal microflora of livestock, a feed composition for improving the intestinal microflora of livestock, a method for preparing the same, a method for improving the intestinal microflora of livestock, a method for proliferating the intestinal lactic acid bacteria of livestock, and a method for promoting the health of livestock.

### [Background Art]

As a problem in livestock breeding, a decrease in the growth of livestock due to various stresses occurring before birth of livestock or during the juvenile stage after birth is exemplified. The body weight of livestock is directly connected to sales and is commercially important, and therefore, such a problem is serious for livestock farmers.

For example, pigs are born in a state where an immune system or a gastrointestinal system is immature, and are liable to be affected by various infectious diseases, diarrhea, or the like. Further, in piglets, due to a stress or the like when switching to solid feed from mother's milk at the weaning stage, the internal organs such as a gastrointestinal tract are weakened, and due to a decrease in feed intake or a remarkable decrease in digestion and absorption of nutritional components, and disturbance of intestinal microflora or a decrease in immunocompetence, the piglets are liable to be infected with pathogens such as bacteria or protozoa. As a result, a decrease in productivity such as the development of diseases or a decrease in growth (body weight gain) and the effect on the quality of products have become problems.

In dealing with the above problems, prevention of the decrease in productivity by increasing lactic acid bacteria (the genus Lactobacillus) or bifidobacteria (the genus Bifidobacterium) that are useful bacteria in the intestinal microflora so as to improve the immune function or the digestive physiological function is considered. For example, Patent Document 1 proposes a preventive or therapeutic agent for diarrhea of livestock containing an enzymatic digest of cell walls of Bifidobacterium thermophilum as an active ingredient.

### [Citation List]

### [Patent Literature]

[PTL 1] JP-A-S56-108717

### [Summary of Invention]

### [Technical Problem]

The invention described in PTL 1 is to improve the intestinal microflora by culturing useful bacteria which originally live in the intestine of livestock, extracting only an active ingredient, and returning it to the host again so as to increase the useful bacteria in the intestine of the livestock. The bifidobacteria used here are obligate anaerobic bacteria, and in the culture of bacterial cells to serve as a raw material, an anaerobic operation is needed, and therefore, the operation is complicated, and moreover, in the growth, an expensive raw material such as a vitamin is needed, and thus, the invention has a disadvantage that the yield of bacterial cells is low, but the cost is high.

Accordingly, an object of the present invention is to improve the intestinal microflora of livestock more easily.

### [Solution to Problem]

The present inventors made intensive studies for solving the above problems, and surprisingly found that by supplying arginine and tannin in combination to livestock, the intestinal microflora of livestock can be improved, and thus completed the present invention.

That is, the present invention is as follows.
1. An agent for improving the intestinal microflora of livestock, containing arginine and tannin as active ingredients.
2. The agent for improving the intestinal microflora of livestock according to the above 1, further containing at least one type of amino acid selected from the group consisting of valine, leucine, isoleucine, cystine, glutamine, and threonine.
3. The agent for improving the intestinal microflora of livestock according to the above 1 or 2, wherein the tannin is derived from a grape or tea extract.
4. The agent for improving the intestinal microflora of livestock according to any one of the above 1 to 3, wherein the arginine is contained in an amount of 25.0 to 55.0 mass% in terms of free form with respect to the total amount of the agent.
5. The agent for improving the intestinal microflora of livestock according to any one of the above 1 to 4, wherein the tannin is contained in an amount of 1.0 to 10.0 mass% with respect to the total amount of the agent.
6. The agent for improving the intestinal microflora of livestock according to any one of the above 1 to 5, wherein the agent is to be added to raw materials for animal feed so as to contain the arginine in an amount of 0.25 to 1.1 g in terms of free form per kg of a livestock feed.
7. The agent for improving the intestinal microflora of livestock according to any one of the above 1 to 6, wherein the agent is to be added to raw materials for animal feed so as to contain the tannin in an amount of 0.01 to 0.2 g per kg of a livestock feed.
8. The agent for improving the intestinal microflora of livestock according to any one of the above 1 to 7, wherein the content ratio of the arginine to the tannin is from 2.5:1 to 55.0:1 on a mass basis.
9. The agent for improving the intestinal microflora of livestock according to any one of the above 1 to 8, wherein the livestock is monogastric livestock.
10. The agent for improving the intestinal microflora of livestock according to any one of the above 1 to 9, wherein the livestock is a pig at the weaning stage.
11. The agent for improving the intestinal microflora of livestock according to any one of the above 1 to 10, wherein the improvement of the intestinal microflora of livestock is the proliferation of the intestinal lactic acid bacteria of the livestock.
12. A feed composition for improving the intestinal microflora of livestock, containing the agent for improving the intestinal microflora of livestock according to any one of the above 1 to 11 and raw materials for animal feed.
13. A method for preparing a feed composition for improving the intestinal microflora of livestock, including a step of adding the agent for improving the intestinal microflora of livestock according to any one of the above 1 to 11 to raw materials for animal feed.
14. A method for improving the intestinal microflora of livestock, for proliferating the intestinal lactic acid bacteria of livestock, or for promoting the health of livestock, including supplying the agent for improving the intestinal microflora of livestock according to any one of the above 1 to 11 to livestock.
15. A method for improving the intestinal microflora of livestock, including adding the agent for improving the intestinal microflora of livestock according to any one of the above 1 to 11 to raw materials for animal feed, and supplying the resulting livestock feed composition to livestock.
17. A method for improving the intestinal microflora of livestock, including supplying the agent for improving the intestinal microflora of livestock according to any one of the above 1 to 11 to livestock.
18. A method for proliferating the intestinal lactic acid bacteria of livestock, including supplying the agent for improving the intestinal microflora of livestock according to any one of the above 1 to 11 to livestock.
19. A method for promoting the health of livestock, including supplying the agent for improving the intestinal microflora of livestock according to any one of the above 1 to 11 to livestock.

### [Advantage Effects of Invention]

The agent for improving the intestinal microflora of livestock of an embodiment of the present invention contains arginine and tannin as active ingredients, and therefore, by supplying the agent to livestock, the intestinal microflora of the livestock can be improved. It is considered that by doing this, the health of the livestock can be promoted, and as a result, the decrease in body weight gain of the livestock can be suppressed.

### [Brief Description of Drawings]

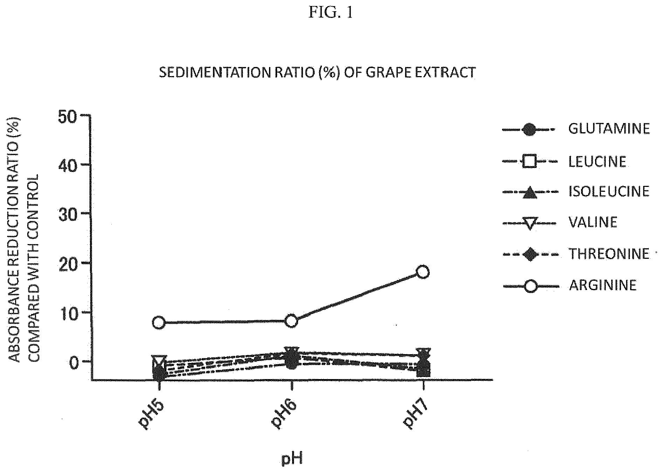

[Fig. 1] Fig. 1 is a graph showing the results of a test for binding affinity between arginine and tannin in Test Example 1-1.

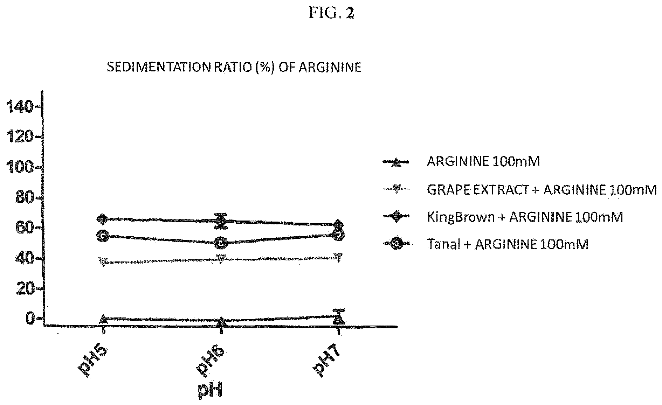

[Fig. 2] Fig. 2 is a graph showing the results of a test for binding affinity between arginine and tannin in Test Example 1-2.

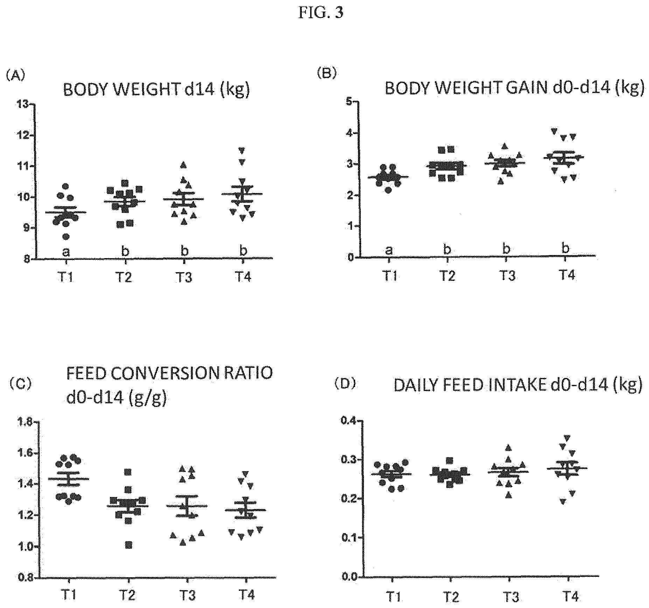

[Fig. 3] Fig. 3 shows graphs indicating the body weight of pigs for each feed group on day 14 (d14) after start of a test (A), the body weight gain of pigs for each feed group until day 14 after start of the test (B), the feed conversion ratio for each feed group until day 14 after start of the test (C), and the daily feed intake until day 14 after start of the test (D).

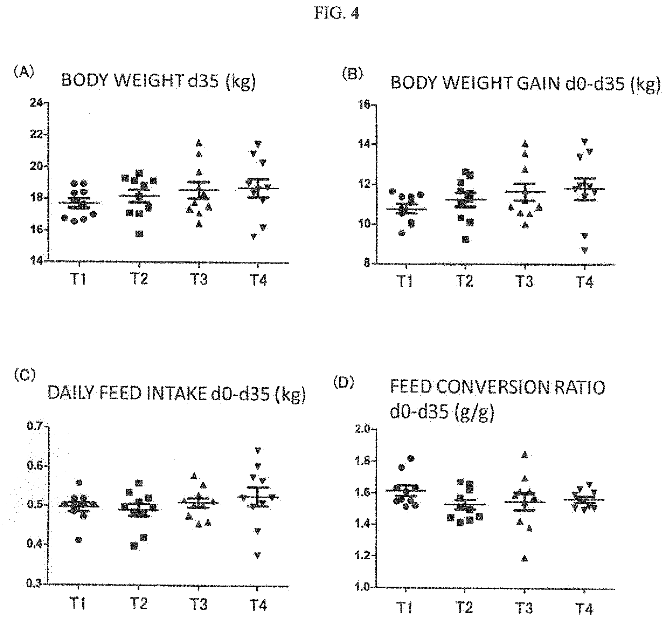

[Fig. 4] Fig. 4 shows graphs indicating the body weight of pigs for each feed group on day 35 (d35) after start of a test (A), the body weight gain of pigs for each feed group until day 35 after start of the test (B), the daily feed intake until day 35 after start of the test (C), and the feed conversion ratio for each feed group until day 35 after start of the test (D).

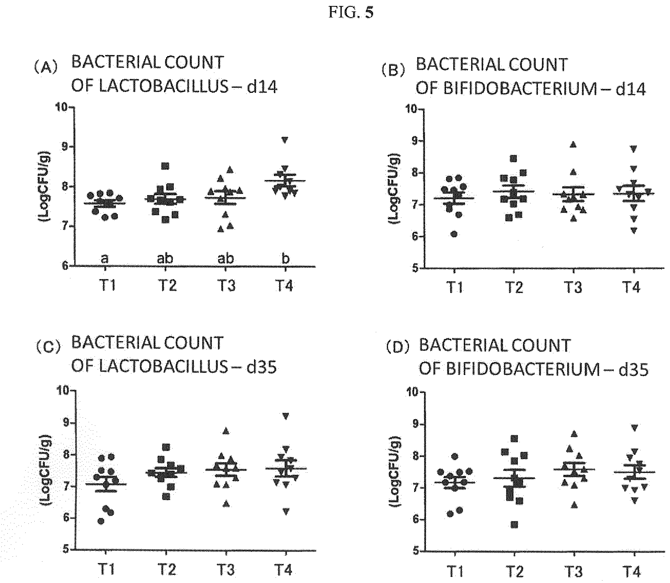

[Fig. 5] Fig. 5 shows graphs indicating the bacterial count of Lactobacillus for each feed group on day 14 (d14) after start of a test (A), the bacterial count of Bifidobacterium for each feed group on day 14 after start of a test (B), the bacterial count of Lactobacillus for each feed group on day 35 (d35) after start of the test (C), and the bacterial count of Bifidobacterium for each feed group on day 35 after start of the test (D).

### [Description of Embodiments]

Hereinafter, specific embodiments of the present invention will be described in detail, however, the invention is by no means limited to the following embodiments, and can be carried out by making appropriate changes within the scope of the object of the invention.

The "improving" as used herein means alleviating, improving, or promoting recovery from aggravated conditions or symptoms. Specifically, the "improving" the intestinal microflora of livestock means alleviating, improving, or promoting recovery from conditions or symptoms in which the bacterial count of intestinal microflora present in the intestine of livestock and the proportions of bacterial strains are aggravated.

The agent for improving the intestinal microflora of livestock (hereinafter also referred to as "improving agent" or simply "agent") of this embodiment contains arginine and tannin as active ingredients. The present inventors found that by allowing livestock to ingest arginine and tannin in combination, the intestinal microflora of the livestock is improved. Specifically, as shown in the below-mentioned Examples, by allowing livestock to ingest arginine and tannin in combination, lactic acid bacteria in the intestine of the livestock could be proliferated. Then, such an effect was higher as compared with the case where livestock was allowed to ingest arginine alone or tannin alone. The reason why the intestinal microflora of livestock can be improved by combining arginine and tannin in this manner has not been clarified, but is considered to be because when livestock ingest both arginine and tannin, as shown in the below-mentioned Examples, arginine and tannin forms a complex in the body of the livestock, and therefore reach the intestinal tract without being degraded, and both arginine and tannin are easily absorbed in the intestine.

Arginine contained in the improving agent of this embodiment may be contained in either form of free arginine or non-free arginine that forms a peptide.

Further, the free arginine or the peptide may be or may not be a salt. Examples of the salt of the free arginine or the peptide include a salt with an inorganic base, a salt with an inorganic acid, a salt with an organic acid, and a salt with an organic base. Examples of the salt with an inorganic base include salts of alkali metals such as sodium, potassium, and lithium, salts of alkaline earth metals such as calcium and magnesium, and ammonium salts. Examples of the salt with an inorganic acid include salts with a halide acid (for example, hydrochloric acid), sulfuric acid, nitric acid, and phosphoric acid. Examples of the salt with an organic acid include salts with formic acid, acetic acid, propionic acid, oxalic acid, succinic acid, maleic acid, fumaric acid, citric acid, and the like. Examples of the salt with an organic base include a nucleotide (for example, a purine derivative and a pyrimidine derivative) and an alkaloid. The free arginine and the peptide also may be or may not be a hydrate or a solvate.

It is preferred that the content of arginine is 25.0 mass% or more in terms of free form with respect to the total amount of the agent. When the content of arginine is 25.0 mass% or more, the amount necessary for obtaining the effect of the present invention can be supplied. The content of arginine is more preferably 30.0 mass% or more, and further more preferably 35 mass% or more.

The content of arginine is preferably 55.0 mass% or less in terms of free form with respect to the total amount of the agent. When the content of arginine is 55.0 mass% or less, an adverse effect of excessive addition thereof can be prevented. The content of arginine is more preferably 50.0 mass% or less, and further more preferably 45.0 mass% or less.

Further, the content of arginine is preferably from 25.0 to 55.0 mass%, more preferably from 30.0 to 50.0 mass%, and further more preferably from 35.0 to 45.0 mass% in terms of free form with respect to the total amount of the agent.

Further, it is preferred that the improving agent of this embodiment is added to raw materials for animal feed so as to contain arginine in an amount of 0.25 g or more in terms of free form per kg of a livestock feed. This is because by adding the agent to raw materials for animal feed so as to contain arginine in an amount of 0.25 g or more in terms of free form per kg of a livestock feed, an amount necessary for obtaining the effect can be supplied. The amount is more preferably 0.30 g or more, and further more preferably 0.35 g or more.

It is preferred that the improving agent of this embodiment is added to raw materials for animal feed so as to contain arginine in an amount of 1.1 g or less in terms of free form per kg of a livestock feed. This is because by adding the agent to raw materials for animal feed so as to contain arginine in an amount of 1.1 g or less in terms of free form per kg of a livestock feed, an adverse effect of excessive addition thereof can be prevented. The amount is more preferably 1.0 g or less, and further more preferably 0.9 g or less.

Further, it is preferred that the improving agent of this embodiment is used for being added to raw materials for animal feed so as to contain arginine in an amount of 0.25 to 1.1 g in terms of free form per kg of a livestock feed, and arginine is contained more preferably in an amount of 0.30 to 1.0 g, and further more preferably in an amount of 0.35 to 0.9 g.

Here, the raw materials for animal feed means feed before the improving agent according to an embodiment of the present invention is added, that is, it means feed to which the improving agent is to be added.

Further, the livestock feed means feed after the improving agent according to an embodiment of the present invention is added, that is, it means the entire feed in which the raw materials for animal feed and the improving agent are put together.

Further, the content of arginine (in terms of free form) in the improving agent can be measured using, for example, an amino acid analyzer (High-Speed Amino Acid Analyzer LA8080 AminoSAAYA, manufactured by Hitachi High-Tech Science Corporation or LC/MS Ultra Fast Amino Acid Analysis System, UF-Amino Station manufactured by Shimadzu Corporation).

Tannin contained in the improving agent of this embodiment is a natural product extracted from plant trunks, skins, leaves, fruits, etc., and is a general name of polyphenolic compounds. Tannin includes pyrogallol-type hydrolysable tannin and catechol-based condensed tannin. As a plant from which tannin is derived, grapes, tea, persimmons, cacaos, Citrus junos, potatoes, apples, blueberries, bananas, chestnut skins, tamarind, mimosa, sumac gallnut, and the like are exemplified, but the plant is not particularly limited thereto. In this embodiment, an extract from a plant containing tannin can be used.

Above all, tannin derived from a grape or tea extract is preferred, and tannin derived from a grape extract is more preferred.

In the improving agent of this embodiment, tannin may be used as a powder by being dried after extraction from a raw material, or a solution obtained by dissolving tannin in a powder form in water may be used as a tannin aqueous solution, or a liquid extracted from a raw material may be used as a tannin aqueous solution as it is.

In the raw material of tannin (for example, grapes, tea or a hot water extract), various types of sugars, organic acids, amino acids, and the like are contained other than tannin, and therefore, it is preferred to use a material obtained by performing purification as needed so as to remove such impurities.

The content of tannin is preferably 1.0 mass% or more with respect to the total amount of the agent. When the content of tannin is 1.0 mass% or more, the amount necessary for obtaining the effect of the present invention can be supplied. The content of tannin is more preferably 1.2 mass% or more, and further more preferably 1.3 mass% or more.

The content of tannin is preferably 10.0 mass% or less with respect to the total amount of the agent. When the content of tannin is 10.0 mass% or less, a decrease in palatability due to an excess of tannin is prevented. The content of tannin is more preferably 5.0 mass% or less, and further more preferably 3.5 mass% or less.

Further, the content of tannin is preferably from 1.0 to 10.0 mass%, more preferably from 1.2 to 5.0 mass%, and further more preferably from 1.3 to 3.5 mass% with respect to the total amount of the agent.

Further, it is preferred that the improving agent of this embodiment is added to raw materials for animal feed so as to contain tannin in an amount of 0.01 g or more per kg of a livestock feed. This is because by adding the agent to raw materials for animal feed so as to contain tannin in an amount of 0.01 g or more per kg of a livestock feed, an amount necessary for obtaining the effect of the present invention can be supplied. The amount is more preferably 0.012 g or more, and further more preferably 0.014 g or more.

It is preferred that the improving agent of this embodiment is added to raw materials for animal feed so as to contain tannin in an amount of 0.2 g or less per kg of a livestock feed. By adding the agent to raw materials for animal feed so as to contain tannin in an amount of 0.2 g or less per kg of a livestock feed, a decrease in palatability due to an excess of tannin is prevented. The amount is more preferably 0.10 g or less, and further more preferably 0.07 g or less.

Further, it is preferred that the improving agent of this embodiment is added to raw materials for animal feed so as to contain tannin in an amount of 0.01 to 0.2 g per kg of a livestock feed, and tannin is more preferably contained in an amount of 0.012 to 0.10 g, and further more preferably contained in an amount of 0.014 to 0.07 g.

The content of tannin in the improving agent of this embodiment can be measured by, for example, a Folin-Denis method. The Folin-Denis method is absorption spectrophotometry using a Folin reagent (phenol reagent), and is adopted as an analysis method of tannin in wine or distilled liquor in the Official Methods of Analysis of AOAC International (AOAC Method 952. 03, 955. 25) (Takeshi Kuribayashi, General Industrial Technology Center, Food Technology Department, Processed Food Group, http://www.gitc.pref.nagano.lg.jp/pdf/gijutujoho275.pdf).

In the improving agent of this embodiment, the content ratio of arginine (in terms of free form) to tannin is preferably from 2.5:1 to 55.0:1 on a mass basis. This is because when the content ratio of arginine to tannin is "2.5:1" or more on a mass basis, a shortage of arginine can be prevented, and when the content ratio is "55.0:1" or less, an excess of arginine is prevented. This is because when the content ratio of arginine to tannin is "(2.5 or more):1" on a mass basis, a shortage of arginine can be prevented, and when the content ratio is "(55.0 or less): 1", an excess of arginine is prevented. The content ratio is more preferably from 3:1 to 30:1, and further more preferably from 4:1 to 25:1.

It is preferred that the improving agent of this embodiment further contains at least one type of amino acid selected from the group consisting of valine, leucine, isoleucine, cystine, glutamine, and threonine in addition to arginine. By containing such an amino acid, a further effect can be expected.

It is more preferred that the improving agent of this embodiment contains at least one type of amino acid selected from the group consisting of valine, leucine, isoleucine, and cystine.

The above-mentioned amino acid may also be contained in either form of a free amino acid or a non-free amino acid that forms a peptide, in the same way as arginine. Further, the free amino acid or the peptide may be or may not be a salt. Further, it may be or may not be a hydrate or a solvate.

When the improving agent of this embodiment contains at least one type of amino acid selected from the group consisting of valine, leucine, isoleucine, cystine, glutamine, and threonine, it is preferred to add the agent to raw materials for animal feed so as to contain such an amino acid in an amount of 0.5 g or more in terms of free form per kg of a livestock feed. This is because by adding the agent to raw materials for animal feed so as to contain the amino acid in an amount of 0.5 g or more in terms of free form per kg of a livestock feed, an amount necessary for obtaining the effect of the present invention can be supplied. The amount is more preferably 0.7 g or more, and further more preferably 1 g or more.

It is preferred that the improving agent of this embodiment is added to raw materials for animal feed so as to contain the amino acid in an amount of 3 g or less in terms of free form per kg of a livestock feed. This is because by adding the agent to raw materials for animal feed so as to contain the amino acid in an amount of 3 g or less in terms of free form per kg of a livestock feed, a decrease in palatability due to an excess of the amino acid is prevented. The amount is more preferably 2.5 g or less, and further more preferably 2 g or less.

Further, it is preferred that the improving agent of this embodiment is added to raw materials for animal feed so as to contain the amino acid in an amount of 0.5 to 3 g in terms of free form per kg of a livestock feed, and the amino acid is more preferably contained in an amount of 0.7 to 2.5 g, and further more preferably contained in an amount of 1 to 2 g.

Further, it is preferred that the improving agent of this embodiment is added to raw materials for animal feed so as to contain valine in an amount of 0.038 g or more per kg of a livestock feed. This is because by adding the agent to raw materials for animal feed so as to contain valine in an amount of 0.038 g or more per kg of a livestock feed, an amount necessary for obtaining the effect of the present invention can be supplied. The amount is more preferably 0.056 g or more, and further more preferably 0.075 g or more.

It is preferred that the improving agent of this embodiment is added to raw materials for animal feed so as to contain valine in an amount of 0.45 g or less per kg of a livestock feed. By adding the agent to raw materials for animal feed so as to contain valine in an amount of 0.45 g or less per kg of a livestock feed, a decrease in palatability due to an excess of valine is prevented. The amount is more preferably 0.38 g or less, and further more preferably 0.3 g or less.

Further, it is preferred that the improving agent of this embodiment is added to raw materials for animal feed so as to contain leucine in an amount of 0.075 g or more per kg of a livestock feed. This is because by adding the agent to raw materials for animal feed so as to contain leucine in an amount of 0.075 g or more per kg of a livestock feed, an amount necessary for obtaining the effect of the present invention can be supplied. The amount is more preferably 0.11 g or more, and further more preferably 0.15 g or more.

It is preferred that the improving agent of this embodiment is added to raw materials for animal feed so as to contain leucine in an amount of 0.90 g or less per kg of a livestock feed. By adding the agent to raw materials for animal feed so as to contain leucine in an amount of 0.90 g or less per kg of a livestock feed, a decrease in palatability due to an excess of leucine is prevented. The amount is more preferably 0.75 g or less, and further more preferably 0.6 g or less.

Further, it is preferred that the improving agent of this embodiment is added to raw materials for animal feed so as to contain isoleucine in an amount of 0.038 g or more per kg of a livestock feed. This is because by adding the agent to raw materials for animal feed so as to contain isoleucine in an amount of 0.038 g or more per kg of a livestock feed, an amount necessary for obtaining the effect of the present invention can be supplied. The amount is more preferably 0.056 g or more, and further more preferably 0.075 g or more.

It is preferred that the improving agent of this embodiment is added to raw materials for animal feed so as to contain isoleucine in an amount of 0.45 g or less per kg of a livestock feed. By adding the agent to raw materials for animal feed so as to contain isoleucine in an amount of 0.45 g or less per kg of a livestock feed, a decrease in palatability due to an excess of isoleucine is prevented. The amount is more preferably 0.38 g or less, and further more preferably 0.3 g or less.

Further, it is preferred that the improving agent of this embodiment is added to raw materials for animal feed so as to contain cystine in an amount of 0.05 g or more per kg of a livestock feed. This is because by adding the agent to raw materials for animal feed so as to contain cystine in an amount of 0.05 g or more per kg of a livestock feed, an amount necessary for obtaining the effect of the present invention can be supplied. The amount is more preferably 0.075 g or more, and further more preferably 0.1 g or more.

It is preferred that the improving agent of this embodiment is added to raw materials for animal feed so as to contain cystine in an amount of 0.75 g or less per kg of a livestock feed. By adding the agent to raw materials for animal feed so as to contain cystine in an amount of 0.75 g or less per kg of a livestock feed, a decrease in palatability due to an excess of cystine is prevented. The amount is more preferably 0.63 g or less, and further more preferably 0.5 g or less.

It is preferred that the improving agent of this embodiment is added to raw materials for animal feed so as to contain glutamine in an amount of 0.15 g or less per kg of a livestock feed. By adding the agent to raw materials for animal feed so as to contain glutamine in an amount of 0.15 g or less per kg of a livestock feed, a decrease in palatability due to an excess of glutamine is prevented. The amount is more preferably 0.13 g or less, and further more preferably 0.1 g or less.

The improving agent of this embodiment is preferably continuously supplied for 15 days or more, more preferably for 21 days or more, and further more preferably for 35 days or more. Further, although the upper limit is not particularly limited, for example, the agent is continuously supplied for 60 days or less. In addition, the agent is administered preferably in a period from 0 to 21 days after weaning, and more preferably in a period from 0 to 35 days after weaning.

In the improving agent of this embodiment, an excipient, an expander, a nutrition reinforcing agent, or another arbitrary component can also be blended as long as the effect of the present invention is not impaired. As the excipient, for example, a cellulose derivative such as carboxymethyl cellulose is exemplified, as the expander, for example, dextrin, starch, and the like are exemplified, as the nutrition reinforcing agent, for example, vitamins and minerals are exemplified, and as another arbitrary component, for example, an enzyme agent and the like are exemplified.

The livestock to which the improving agent of this embodiment can be applied is not particularly limited, but in order to avoid the possibility of degradation in the first stomach of a ruminant, it is preferably monogastric livestock. Examples of the monogastric livestock include a pig, a domestic fowl, a horse, and the like. The livestock is more preferably a pig or a domestic fowl.

Further, in the case of a mammal, the agent is preferably applied at the weaning stage. The weaning stage as used herein indicates a stage at which the digestive physiology of livestock has reached a mature state although it depends on the growth state of livestock, and in the case of a pig, it refers to one which has passed the age of 21 to 28 days.

Further, examples of the domestic fowl include a chicken, a quail, a turkey, a duck, a goose, and the like, and the domestic fowl is more preferably a chicken.

By supplying the improving agent of this embodiment to livestock, the intestinal microflora of the livestock can be improved. Further, the intestinal lactic acid bacteria of livestock can be proliferated. Then, by improving the intestinal microflora of the livestock, the digestion and nutrient absorption function of the intestine can be optimized, and as a result, the health of the livestock can be promoted.

The improving agent of this embodiment may be supplied to livestock after obtaining a livestock feed composition by adding the agent to raw materials for animal feed as described below, or the improving agent may be directly supplied to livestock.

As another embodiment of the present invention, a feed composition for improving the intestinal microflora of livestock (hereinafter also simply referred to as "feed composition") prepared by adding the above-mentioned improving agent to raw materials for animal feed is provided.

The raw materials for animal feed is not particularly limited, but raw materials, for example, grains such as corn, barley, wheat, rye, sorghum, soybeans, and soybean flour, soybean oil cakes, soy protein, oils and fats, skim milk, fish meal, meat and bone meal, blood meal, plasma protein, whey, rice bran, wheat bran, saccharides such as sugar or other sweeteners, minerals, vitamins, table salt, and the like can be used alone or in combination.

As another embodiment of the present invention, a method for preparing a feed composition for improving the intestinal microflora of livestock including a step of adding the above-mentioned improving agent to raw materials for animal feed is provided. A method of adding the improving agent to raw materials for animal feed is not particularly limited, and a conventionally known method can be used. For example, addition to a mash, or pelletization is exemplified. Further, as the raw materials for animal feed, those described above can be used.

As another embodiment of the present invention, a method for improving the intestinal microflora of livestock including supplying the above-mentioned improving agent to livestock is provided. In such a method, the improving agent itself may be directly supplied to livestock without adding the agent to raw materials for animal feed. The amount of the improving agent to be supplied to livestock, the number of days for which the agent is supplied, and the like are as described above. By supplying the improving agent to livestock, the intestinal microflora of the livestock can be improved.

As another embodiment of the present invention, a method for proliferating the intestinal lactic acid bacteria of livestock including supplying the above-mentioned improving agent to livestock is provided. In such a method, the improving agent itself may be directly supplied to livestock without adding the agent to raw materials for animal feed. The amount of the improving agent to be supplied to livestock, the number of days for which the agent is supplied, and the like are as described above. By supplying the improving agent to livestock, the intestinal lactic acid bacteria of the livestock can be proliferated.

As another embodiment of the present invention, a method for improving the intestinal microflora of livestock including adding the above-mentioned improving agent to raw materials for animal feed, and supplying the resulting livestock feed composition to livestock is provided. As the raw materials for animal feed, those described above can be used. Further, the amount of the feed composition to be supplied to livestock, the number of days for which the agent is supplied, and the like are also as described above. By supplying the livestock feed composition to livestock, the intestinal microflora of the livestock can be improved.

As another embodiment of the present invention, a method for promoting the health of livestock including supplying the above-mentioned improving agent to livestock is provided. The amount of the improving agent to be supplied to livestock, the number of days for which the agent is supplied, and the like are as described above. By supplying the improving agent to livestock, the intestinal microflora of the livestock can be improved, and further, the digestion and nutrient absorption function of the intestine can be optimized, and as a result, the health of the livestock can be promoted.

As the intestinal microflora in the present invention, for example, lactic acid bacteria and bifidobacteria are exemplified. As the lactic acid bacteria, for example, bacteria classified into any of the genus Lactobacillus, the genus Streptococcus, the genus Leuconostoc, the genus Pediococcus, the genus Enterococcus, and the genus Lactococcus are exemplified.

### [Examples]

Hereinafter, the present invention will be further described with reference to Examples, however, the invention is not limited to the following examples. The measurement of the respective items in Examples was carried out by the following methods.

### [Initial Body Weight, Final Body Weight, and Body Weight Gain]

The body weight of each individual was measured using a weighing scale for livestock when starting and finishing a test, and an arithmetic mean was calculated for each group.

### [Daily Feed Intake (DFI)]

The intake was measured from a difference between the feeding amount and the residual feed amount for a given number of days. The intake was divided by the number of days and the number of pigs, whereby the daily average feed intake was calculated for each group.

### [Average Daily Body Weight Gain (ADG)]

The body weight gain for a given number of days was divided by the number of days, whereby the average daily body weight gain was calculated.

### [Feed Conversion Ratio (FCR)]

The daily feed intake was divided by the average daily body weight gain, whereby the feed conversion ratio was calculated.

### [Survival Rate]

The number of surviving pigs after a given number of days passed with respect to the total number of pigs was calculated, and the result was defined as the survival rate.

### [Bacterial Count of Lactic Acid Bacteria]

The feces of pigs were collected from each pen after a given number of days passed, and the bacterial count of lactic acid bacteria (Lactobacillus) was measured.

### Test Example 1: Test for Binding Affinity between Arginine and Tannin

### (Test Example 1-1)

In this test example, the binding affinity between arginine and tannin was confirmed according to the following procedure.
1. A 1 mg/mL grape extract (tannin content: 68.5%) and 100 mM AA (any one type of Leu, Arg, BCAA, Thr, and Gln) were mixed, and allowed to react under the following conditions 1) to 3).
   1) a reaction at pH 5 for 2 hours
   2) a reaction at pH 6 for 2 hours
   3) a reaction at pH 7 for 2 hours
2. 200 µL of each of the solutions obtained by the reactions under the above conditions 1) to 3) was dispensed into a 96-well plate (UV-STAR plate, manufactured by Greiner Bio-One) in 3 separate parts.
3. The absorbance at 280 nm was measured using a spectrophotometer (Spectra Max, manufactured by Molecular Devices, LLC). The absorbance of each solution (AA + grape extract) was represented by a relative value to the absorbance of a solution containing only the grape extract (control). The results are shown in Fig. 1.

As is also apparent from the results of Fig. 1, particularly in the case where arginine among the amino acids was mixed with the grape extract, the highest absorbance was obtained, and this indicates that the binding affinity between arginine and the grape extract (tannin) is high.

### (Test Example 1-2)

In this test example, the binding affinity between arginine and tannin (a grape extract, TANAL 01 or KingBrown (registered trademark)) was confirmed according to the following procedure.
1. 1 mg/mL tannin (a grape extract, either one of TANAL 01 (manufactured by Ajinomoto NaturalSpecialities) and KingBrown (manufactured by King Tree Company) and 100 mM AA (Arg) were mixed, and allowed to react under the following conditions 1) to 3).
   1) a reaction at pH 5 for 2 hours
   2) a reaction at pH 6 for 2 hours
   3) a reaction at pH 7 for 2 hours
2. In 1 mL of the supernatant of each of the solutions obtained by the reactions under the above conditions 1) to 3), 0.75 mL of an acetate buffer solution (pH 5.6) and 0.4 mL of ninhydrin (3.5 mg/L) were mixed, and the resulting mixture was incubated at 37°C for 25 minutes.
3. The absorbance at 570 nm was measured using a spectrophotometer (Spectra Max, manufactured by Molecular Devices, LLC). The absorbance of each solution (Arg + tannin) was represented by a relative value to the absorbance of a solution containing only tannin (control). The results are shown in Fig. 2.

As is also apparent from the results of Fig. 2, in the case of arginine, a high absorbance was obtained even when it was mixed with any of the grape extract, TANAL 01 and KingBrown, and this indicates that the binding affinity between arginine and tannin is high.

### Test Example 2: Test for Supplying to Pigs at Weaning Stage

As the test animals, the total of 240 crossbred (Landrace × Large White × Duroc) weaned piglets at the average age of 25 days with an average body weight of 6.92 kg were used. These weaned piglets were grouped into four feed supply modes: T1 to T4. Each test group was composed of 10 pens, each of which consisted of 6 piglets ((3 male piglets + 3 female piglets)/pen), and the piglets were randomly allocated to the pens. The size of each pen was set to 1.5 m × 2.0 m.

The feed supply modes in the test groups T1 to T4 are shown in Table 1.

### [Table 1]

**Table 1**

| Test group | d0-d14 | d14-d35 |
|---|---|---|
| T1 | Pre-starter feed | Common starter feed |
| T2 | T1 + 0.1% AA mix | |
| T3 | T1 + 85 ppm grape seed extract + 15 ppm grape skin extract | |
| T4 | T1 + 0.1% AA mix + 85 ppm grape seed extract + 15 ppm grape skin extract | |

| | | |
|---|---|---|
| Grape seed extract (containing 75% or more tannin) Grape skin extract (containing 30% or more tannin) | | |

▪ Test group T1: A basal diet (pre-starter feed: the composition is shown in Table 3) was supplied to the piglets for the first two weeks (d0 to d14), and a common starter feed (the composition is shown in Table 3) was supplied to the piglets for the latter three weeks (d14 to d35).
▪ Test group T2: A livestock feed in which 0.1 wt% AA mix (the composition is shown in Table 2) was added to a basal diet (pre-starter feed) was prepared and supplied to the piglets for the first two weeks (d0 to d14). A common starter feed was supplied to the piglets for the latter three weeks (d14 to d35).
▪ Test group T3: A livestock feed in which an 85 wt ppm grape seed extract and a 15 wt ppm grape skin extract were added to a basal diet (pre-starter feed) was prepared and supplied to the piglets for the first two weeks (d0 to d14). A common starter feed was supplied to the piglets for the latter three weeks (d14 to d35).
▪ Test group T4: A livestock feed in which an agent containing 0.1 wt% AA mix, an 85 wt ppm grape seed extract, and a 15 wt ppm grape skin extract was added to a basal diet (pre-starter feed) was prepared and supplied to the piglets for the first two weeks (d0 to d14). A common starter feed was supplied to the piglets for the latter three weeks (d14 to d35).

Note that in the above agent, arginine was contained in an amount of 37.9 wt% in terms of free form, and tannin in an amount of 9.1 wt%. Further, in 1 kg of the livestock feed, arginine was contained in an amount of 0.42 g in terms of free form, and tannin was contained in an amount of 0.1 g. Further, the livestock feed was supplied ad libitum.

The composition of the AA mix is shown in Table 2

The compositions of the basal diet (pre-starter feed) and the common starter feed are shown in Table 3. Note that the symbol "-" in Table 3 denotes that the raw material is not contained.

### [Table 2]

**Table 2**

| (wt%) | |
|---|---|
| arginine | 41.7 |
| valine | 8.3 |
| leucine | 16.7 |
| isoleucine | 8.3 |
| cystine | 25.0 |
| Total | 100 |

### [Table 3]

**Table 3**

| Raw material | unit | Pre-starter feed | Common starter feed |
|---|---|---|---|
| Corn | wt% | 30 | 20 |
| Broken rice | wt% | 21.23 | 39.38 |
| Soybean meal (DH) | wt% | 27.41 | 23.67 |
| Sweet whey powder | wt% | 10 | - |
| Wheat bran | wt% | 5 | 11.89 |
| Soybean oil | wt% | 2.65 | 1.1 |
| Limestone | wt% | 1.89 | 1.93 |
| MDCP | wt% | - | 0.35 |
| Salt | wt% | 0.4 | 0.4 |
| Premix | wt% | 0.5 | 0.5 |
| Quantum blue | wt% | 0.01 | 0.01 |
| L-lvsine | wt% | 0.38 | 0.38 |
| DL-methionine | wt% | 0.23 | 0.16 |
| L- Threonine | wt% | 0.2 | 0.17 |
| L-Valine | wt% | 0.06 | 0.01 |
| L- Tryptophan | wt% | 0.06 | 0.04 |
| Total | wt% | 100 | 100 |
| Weight | wt% | 100 | 100 |
| Dry matter | wt% | 88.98 | 88.09 |
| Crude protein | wt% | 20 | 18.67 |
| Crude fat | wt% | 4.85 | 3.05 |
| Crude fibre | wt% | 2.99 | 3.36 |
| Crude ash | wt% | 5.71 | 5.38 |
| Starch | wt% | 36.59 | 45.54 |
| Ca | wt% | 1.1 | 1.1 |
| Phosphorous digestible pig | wt% | 0.301 | 0.3 |
| Na | wt% | 0.231 | 0.167 |
| Electrolyte balance | meq/kg | 206 | 179 |
| NE pig (MJ/kg) | MJ/kg | 10.5 | 10 |
| NE pig (kCal/kg) | kCal/kg | 2509 | 2389 |
| LYS | wt% | 1.356 | 1.222 |
| SID LYS pig | wt% | 1.23 | 1.1 |
| SID THR pig | wt% | 0.799 | 0.715 |
| SID MET pig | wt% | 0.475 | 0.404 |
| SID CYS pig | wt% | 0.263 | 0.256 |
| SID M+C pig | wt% | 0.738 | 0.66 |
| SID TRP pig | wt% | 0.271 | 0.242 |
| SID ILE pig | wt% | 0.727 | 0.662 |
| SID VAL pig | wt% | 0.861 | 0.77 |
| SID LEU pig | wt% | 1.336 | 1.207 |
| SID PHE pig | wt% | 0.848 | 0.776 |
| SID TYR pig | wt% | 0.523 | 0.493 |
| SID P+T pig | wt% | 1.371 | 1.269 |
| SID HIS pig | wt% | 0.498 | 0.459 |
| SID ARG pig | wt% | 1.184 | 1.124 |

With respect to the initial body weight, the final body weight, the body weight gain, the daily feed intake, the average daily body weight gain, the feed conversion ratio, and the survival rate, the results from day 0 to day 14 (d0 to d14) are shown in Table 4 and Fig. 3, the results from day 14 to day 35 (d14 to d35) are shown in Table 5, and the results from day 0 to day 35 (d0 to d35) are shown in Table 6 and Fig. 4.

### [Table 4]

**Table 4 (d0-d14)**

| Test group | Added component | | | Initial body weight | Final body weight | Body weight gain | Daily feed intake (DFI) | Average daily body weight gain (ADG) | Feed conversion ratio (FCR) | Survival rate |
|---|---|---|---|---|---|---|---|---|---|---|
| | AA mix | Grape seed extract | Grape skin extract | | | | | | | |
| | | | | (kg) | (kg) | (kg) | (kg) | (kg) | | (%) |
| T1 | - | - | - | 6.92 | 9.50^{b} | 2.575^{b} | 0.262^{b} | 0.184^{b} | 1.431^{a} | 96.67 |
| T2 | 0.10% | - | - | 6.92 | 9.85^{a} | 2.923^{a} | 0.301^{a} | 0.209^{a} | 1.257^{b} | 95 |
| T3 | - | 85 ppm | 15 ppm | 6.92 | 9.92^{a} | 2.996^{a} | 0.267^{b} | 0.214^{a} | 1.257^{b} | 96.67 |
| T4 | 0.10% | 85 ppm | 15 ppm | 6.92 | 10.07^{a} | 3.146^{a} | 0.275^{ab} | 0.225^{a} | 1.229^{b} | 95 |

| | | | | | | | | | | |
|---|---|---|---|---|---|---|---|---|---|---|
| a, b: Different superscript letters indicate statistically significant differences (P < 0.05) | | | | | | | | | | |

### [Table 5]

**Table 5 (d14-d35)**

| Test group | Added component | | | Initial body weight | Final body weight | Body weight gain | Daily feed intake (DFI) | Average daily body weight gain (ADG) | Feed conversion ratio (FCR) | Survival rate |
|---|---|---|---|---|---|---|---|---|---|---|
| | AA mix | Grape seed extract | Grape skin extract | | | | | | | |
| | | | | (kg) | (kg) | (kg) | (kg) | (kg) | | (%) |
| T1 | - | - | - | 9.50^{b} | 17.72 | 8.22 | 0.659 | 0.392 | 1.682 | 94.67 |
| T2 | 0.10% | - | - | 9.85^{a} | 18.17 | 8.33 | 0.652 | 0.396 | 1.649 | 94.67 |
| T3 | - | 85 ppm | 15 ppm | 9.92^{a} | 18.56 | 8.64 | 0.681 | 0.411 | 1.675 | 96.67 |
| T4 | 0.10% | 85 ppm | 15 ppm | 10.07^{a} | 18.72 | 8.65 | 0.705 | 0.412 | 1.711 | 96 |

| | | | | | | | | | | |
|---|---|---|---|---|---|---|---|---|---|---|
| a, b: Different superscript letters indicate statistically significant differences (P < 0.05) | | | | | | | | | | |

### [Table 6]

**Table 6 (d0-d35)**

| Test group | Added component | | | Initial body weight | Final body weight | Body weight gain | Daily feed intake (DFI) | Average daily body weight gain (ADG) | Feed conversion ratio (FCR) | Survival rate |
|---|---|---|---|---|---|---|---|---|---|---|
| | AA mix | Grape seed extract | Grape skin extract | | | | | | | |
| | | | | (kg) | (kg) | (kg) | (kg) | (kg) | | (%) |
| T1 | - | - | - | 6.92 | 17.72 | 10.8 | 0.497 | 0.309 | 1.612 | 91.67 |
| T2 | 0.10% | - | - | 6.92 | 18.17 | 11.25 | 0.508 | 0.321 | 1.586 | 90 |
| T3 | - | 85 ppm | 15 ppm | 6.92 | 18.56 | 11.64 | 0.508 | 0.332 | 1.544 | 93.33 |
| T4 | 0.10% | 85 ppm | 15 ppm | 6.92 | 18.72 | 11.8 | 0.526 | 0.337 | 1.56 | 91.67 |

Further, with respect to the bacterial count of lactic acid bacteria (Lactobacillus) and the bacterial count of bifidobacteria (Bifidobacterium), the results on day 14 (d14) and on day 35 (d35) are shown in Table 7 and Fig. 5.

### [Table 7]

**Table 7**

| Test group | Added component | | | d14 | | d35 | |
|---|---|---|---|---|---|---|---|
| | AA mix | Grape seed extract | Grape skin extract | Lactobacillus | Bifidobacterium | Lactobacillus | Bifidobacterium |
| | | | | (logCFU/g) | (logCFU/g) | (logCFU/g) | (logCFU/g) |
| T1 | - | - | - | 7.47 | 7.21 | 7.08 | 7.18 |
| T2 | 0.10% | - | - | 7.7 | 7.42 | 7.46 | 7.32 |
| T3 | - | 85 ppm | 15 ppm | 7.74 | 7.33 | 7.56 | 7.6 |
| T4 | 0.10% | 85 ppm | 15 ppm | 7.96 | 7.36 | 7.61 | 7.52 |

It was found that in the test group T4 in which the agent containing both arginine and tannin according to the present invention was supplied, the body weight gain was higher as compared with the test group T1 in which the feed that does not contain either arginine or tannin was supplied, and the test groups T2 and T3 in which the feed obtained by adding the agent containing arginine or tannin alone was supplied, and also the bacterial count of lactic acid bacteria (Lactobacillus) increased.

## Claims

1. An agent for improving the intestinal microflora of livestock, comprising arginine and tannin as active ingredients.

2. The agent for improving the intestinal microflora of livestock according to claim 1, further comprising at least one type of amino acid selected from the group consisting of valine, leucine, isoleucine, cystine, glutamine, and threonine.

3. The agent for improving the intestinal microflora of livestock according to claim 1 or 2, wherein the tannin is derived from a grape or tea extract.

4. The agent for improving the intestinal microflora of livestock according to any one of claims 1 to 3, wherein the arginine is contained in an amount of 25.0 to 55.0 mass% in terms of free form with respect to the total amount of the agent.

5. The agent for improving the intestinal microflora of livestock according to any one of claims 1 to 4, wherein the tannin is contained in an amount of 1.0 to 10.0 mass% with respect to the total amount of the agent.

6. The agent for improving the intestinal microflora of livestock according to any one of claims 1 to 5, wherein the agent is to be added to raw materials for animal feed so as to contain the arginine in an amount of 0.25 to 1.1 g in terms of free form per kg of a livestock feed.

7. The agent for improving the intestinal microflora of livestock according to any one of claims 1 to 6, wherein the agent is to be added to raw materials for animal feed so as to contain the tannin in an amount of 0.01 to 0.2 g per kg of a livestock feed.

8. The agent for improving the intestinal microflora of livestock according to any one of claims 1 to 7, wherein the content ratio of the arginine to the tannin is from 2.5:1 to 55.0:1 on a mass basis.

9. The agent for improving the intestinal microflora of livestock according to any one of claims 1 to 8, wherein the livestock is monogastric livestock.

10. The agent for improving the intestinal microflora of livestock according to any one of claims 1 to 9, wherein the livestock is a pig at the weaning stage.

11. The agent for improving the intestinal microflora of livestock according to any one of claims 1 to 10, wherein the improvement of the intestinal microflora of livestock is the proliferation of the intestinal lactic acid bacteria of the livestock.

12. A feed composition for improving the intestinal microflora of livestock, comprising the agent for improving the intestinal microflora of livestock according to any one of claims 1 to 11 and raw materials for animal feed.

13. A method for preparing a feed composition for improving the intestinal microflora of livestock, comprising a step of adding the agent for improving the intestinal microflora of livestock according to any one of claims 1 to 11 to raw materials for animal feed.

14. A method for improving the intestinal microflora of livestock, for proliferating the intestinal lactic acid bacteria of livestock, or for promoting the health of livestock comprising supplying the agent for improving the intestinal microflora of livestock according to any one of claims 1 to 11 to livestock.

15. A method for improving the intestinal microflora of livestock, comprising adding the agent for improving the intestinal microflora of livestock according to any one of claims 1 to 11 to raw materials for animal feed, and supplying the resulting livestock feed composition to livestock.
